# EUROPEAN PATENT APPLICATION

(11) **EP 4 703 754 A1**
(43) Date of publication of application: **04.03.2026**
(21) Application number: 24275100.6
(22) Date of filing: 28.08.2024
(51) Int. Cl.: G01R 33/38, G01R 33/3815

(54) **MAGNET ARRANGEMENT AND METHOD OF MANUFACTURING**

(71) Applicant: Siemens Healthcare Limited, Camberley, Surrey GU15 3YL (GB)
(72) Inventor: Mallett, Michael, Faringdon, SN7 8EL (GB)
(74) Representative: Siemens Healthineers Patent Attorneys

(57) **Abstract**

The invention relates to a magnet arrangement (11) for a magnetic resonance device (10), configured to provide a magnetic field suitable for use in a magnetic resonance imaging examination, including a magnet coil (31) wound from a tape (32) comprising a superconducting material (35), wherein a width of the tape (32) varies along on a length of the tape (32), and wherein a mass distribution of the superconducting material (35) over a radial cross-section of the magnet coil (31) is dependent on a distribution of a magnetic field strength of the magnetic field over the radial cross-section of the magnet coil (31). The invention further relates to a magnetic resonance device (10) comprising an inventive magnet arrangement (11) and a method of manufacturing a magnet coil (31) of an inventive magnet arrangement (11).

## Description

Independent of the grammatical term usage, individuals with male, female or other gender identities are included within the term.

The current generation of high temperature superconductors (HTS) is manufactured from rare earth barium copper oxide (REBCO) materials, where the rare earth is commonly Yttrium or Gadolinium. The manufacturing process comprises depositing a thin layer of the REBCO material onto a prepared substrate, e. g. a stainless-steel alloy including nickel, but also cobalt, chromium, molybdenum, tungsten, iron, silicon, manganese, carbon, aluminium, titanium, or the like. As a result, a wide tape-like material, typically 10-12mm in width and 0.1-0.2m in thickness is provided. The tape is then usually cut into thinner stripes of about 4 mm width before being provided to an end user. The end user may wind the supplied tape into a coil using, for example, the single or double pancake winding method. Low performance coils can be wound from narrow tape, whereas high performance coils may require the use of a wider tape. The optimal use of the tape is governed by using a tape width appropriate to the maximum operating condition of the magnet coil.

An alternative tape slitting method is used to produce what is known as a Roebel cable. This slitting method involves cutting the wide HTS tape into a shape which allows interleaving of multiple pieces of cut tape to create a wire structure which has better performance under AC magnetic field conditions in comparison to straight or rectangular tapes. However, the manufacturing process of Roebel cables is complex and associated with a significant amount of waste material which must be discarded.

It is an object of the invention to enable a more efficient use of superconducting tape in a superconducting magnet.

This object is achieved by a magnet arrangement, a magnetic resonance device and a method according to the invention. Further advantageous embodiments are specified in the dependent claims.

The inventive magnet arrangement is configured for use in a magnetic resonance device. The magnet arrangement may comprise or represent a main magnet of a magnetic resonance device.

However, the inventive magnet arrangement may also be used in other applications where an economic use of expensive tape is favourable. These applications could include, but are not limited to, magnetic resonance imaging, nuclear magnetic resonance, fusion magnets, accelerator magnets, but also motors and generators comprising a high temperature superconducting material.

It is conceivable that the magnet arrangement comprises one or more magnet coils. In a preferred embodiment, the magnet arrangement comprises at least two magnet coils. The at least two magnet coils may be rotationally symmetric or comprise rotationally symmetric bodies. Preferably, the at least two magnet coils represent cylindrical superconducting coils.

The one or more magnet coils of the magnet arrangement may define a common axis. Preferably, the common axis defined by one or more magnet coils corresponds to a cylinder axis and/or an axis of rotational symmetry of the main magnet and/or the magnet arrangement.

According to an embodiment, the one or more magnet coils are mechanically coupled and/or mechanically connected. Preferably, the one or more magnet coils are mechanically connected in such a way to form a cohesive or coherent structure.

For example, the one or more magnet coils may be integrally bonded. Preferably, at least two magnet coils are integrally bonded to at least one spacer arranged between the at least two magnet coils. For example, an adhesive, particularly an epoxy resin, may be used to bond the at least two magnet coils to opposing sides or faces of the at least one spacer.

The magnet arrangement may comprise a magnet support structure configured to provide mechanical support to the one or more magnet coils. Particularly, the magnet support structure may be configured to maintain a predefined spatial arrangement of at least two magnet coils. According to an embodiment, the magnet support structure is configured to be mechanically connected to a support structure, particularly an outer vacuum chamber, of a magnetic resonance device comprising the inventive magnet arrangement.

The magnet arrangement may comprise a cryogen vessel and/or a thermal shield. It is conceivable that the magnet arrangement is circumferentially enclosed in an outer vacuum chamber of a magnetic resonance device. The vacuum chamber may represent a double-walled hollow cylinder comprising an outer shell and an inner shell. The outer shell and the inner shell of the vacuum chamber may be connected by annular end pieces. The magnet arrangement may be enclosed between the outer shell and the inner shell of the outer vacuum chamber. The inner shell of the outer vacuum chamber may correspond to a patient bore of a magnetic resonance device comprising the inventive magnet arrangement.

The magnet arrangement is configured to provide a magnetic field suitable for use in a magnetic resonance imaging examination. Particularly, the magnet arrangement may be configured to provide a static or main magnetic field (i. e. a B0-field) of the magnetic resonance device. It is conceivable that a maximum magnetic field strength of the magnetic field is between 0.1 and 9 Tesla, particularly 0.5 Tesla, 1.5 Tesla, 3 Tesla, or 7 Tesla.

According to the invention, the magnet arrangement includes a magnet coil wound from a tape comprising a superconducting material.

The tape may comprise a substrate and a superconducting material. For example, the substrate may comprise or consist of a metal alloy and/or a fibre composite. Particularly, the substrate may comprise one or more of a thin-rolled metal alloy, a metal, and a fibre composite. Preferably, the substrate consists of a stainless-steel alloy. The stainless-steel alloy may include nickel, cobalt, chromium, molybdenum, tungsten, iron, silicon, manganese, carbon, aluminium, and/or titanium.

According to a preferred embodiment, the substrate is electrically conductive.

According to an embodiment, the magnet coil may comprise one or more tapes comprising a superconducting material. For example, the magnet coil may comprise a single continuous tape or a plurality of tapes (or segments of tape) which are connected via one or more joints. The superconducting material may be deposited or disposed on the substrate in the form of a layer, multiple layers, or a wire.

In a preferred embodiment of the inventive magnet arrangement, the superconducting material is a high temperature superconducting material.

High temperature superconducting materials may correspond to a class of superconducting materials having a high critical temperature in comparison to low temperature superconducting materials. For example, a high temperature superconducting material may have a critical temperature between 50 K to 150 K. Some high temperature superconducting materials, such as rare-earth barium copper oxides (REBCO), can be produced as long strands or tapes which can be wound into magnet coils for use in fusion devices, magnetic resonance devices, or other applications.

According to an embodiment, the superconducting material comprises a rare earth barium copper oxide (REBCO) material or a combination of such materials, such as may be provided by a number of different yttrium barium copper oxide (YBCO) compounds.

In providing a magnet arrangement comprising a high temperature superconducting material, a temperature level of cooling equipment required for maintaining the magnet coil at a temperature level at or below the critical temperature of the superconducting material may be increased in comparison to conventional low temperature superconducting materials, such as Nb₃Sn, Nb₃Al, and NbTi. Thus, an efficiency of the cooling equipment may favourably be increased and operational costs of the magnetic resonance device comprising the inventive magnet arrangement may be reduced.

According to an embodiment, the magnet coil of the inventive magnet arrangement comprises one or more tapes. Particularly, the magnet coil may comprise a plurality of tapes or a plurality of tape segments. The plurality of tapes may represent cylindrical coils arranged concentrically along an axial direction, particularly a cylinder axis or an axis of rotational symmetry, defined by the magnet coil. However, the plurality of tapes may also be stacked in a radial direction of the magnet coil. It is conceivable that two or more tapes of the plurality of tapes are electrically connected via a joint. For example, the two or more tapes may be electrically connected via a solder joint.

According to the invention, a width of the tape varies along on a length of the tape. For example, the tape may comprise an oblong shape, such as the shape of a stripe, a band, an elongated plate, or a flat bar. Preferably, mechanical properties of the tape are such that the tape can be wound into a spool or a coil without breaking or cracking.

In a preferred embodiment, the tape may have a thickness between 0.1 and 0.2 mm. However, the tape may be thinner or thicker depending on the requirements of the application, such as the magnetic field strength of the magnetic resonance device comprising the inventive magnet arrangement.

A width of the tape may vary between 1 mm and 6 mm along the length of the tape. It is conceivable, however, that sections of the tape have a smaller width or a larger width depending on the requirements of the application.

A mass distribution of the superconducting material over a radial cross-section of the magnet coil is dependent on a distribution of a magnetic field strength of the magnetic field over the radial cross-section of the magnet coil.

In a preferred embodiment, the mass distribution of the superconducting material over the radial cross-section of the magnet coil is correlated with the distribution of the magnetic field strength of the magnetic field over the radial cross-section of the magnet coil. For example, the mass distribution of the superconducting material over the radial cross-section of the magnet coil may correlate with the distribution of the magnetic field strength of the magnetic field over the radial cross-section of the magnet coil in a linear or a non-linear fashion.

It is also conceivable that the mass distribution of the superconducting material over the radial cross-section of the magnet coil corresponds to the distribution of the magnetic field strength of the magnetic field over the radial cross-section of the magnet coil. For example, a mass gradient of the superconducting material over a section of the radial cross-section of the magnet coil may correlate with or correspond to a magnetic field gradient over the section of the radial cross-section of the magnet coil.

In a preferred embodiment, the mass distribution of the superconducting material over the radial cross-section of the magnet coil depends on a variation of the width of the tape along the length of the tape of the magnet coil. Particularly, a locally reduced width of the tape of the magnet coil may cause a locally reduced mass of the superconducting material. Thus, the mass distribution of the superconducting material over the radial cross-section of the magnet coil may depend on and/or or correlate with the varying width of the tape along the length of the tape.

An inventive magnet arrangement may favourably take account of the fact that not all of the superconducting wire inside a typical superconducting magnet requires the same level of performance. For example, the highest performing parts of the magnet coil may be located at axial ends and/or a radially inner surface of the magnet coil. In these locations, the superconducting material may be exposed to higher magnetic field strengths of the background magnetic field generated by the one or more magnet coils of the magnet arrangement. In order to sustain a magnet current in these locations, the amount of superconducting material may be locally increased by increasing the width of the tape. Conversely, the magnetic field strength of the background magnetic field may decrease in a direction towards a geometric centre and/or a radial centre line of the radial cross-section of the magnet coil. In regions with lower magnetic field strengths, the amount of required superconducting material may favourably be reduced by reducing the width the tape, since only a low performance is required to sustain the magnet current.

An inventive magnet arrangement may favourably reduce an amount of superconducting material required for a magnet coil in comparison to conventional superconducting magnets. Thus, costs and/or weight associated with the inventive magnet arrangement may favourably be reduced in comparison to conventional magnet arrangements.

Particularly, an inventive magnet arrangement may favourably allow for a distribution of superconducting material to efficiently match a background magnetic field condition, e. g. a magnetic field strength, a magnetic field orientation, a magnetic field vector, or the like.

According to a preferred embodiment of the inventive magnet arrangement, the mass distribution of the superconducting material over the radial cross-section of the magnet coil is dependent on a vector field of the magnetic field over the radial cross-section of the magnet coil.

For example, the mass distribution of the superconducting material over the radial cross-section of the magnet coil may be correlated with a distribution of an orientation of the magnetic field (i. e. the magnetic field vector) over the radial cross-section of the magnet coil. Particularly, the mass distribution of the superconducting material over the radial cross-section of the magnet coil may correlate with the distribution of the magnetic field orientation over the radial cross-section of the magnet coil in a linear or a non-linear fashion. It is conceivable that the mass of the superconducting material in a section of the radial cross-section of the magnet coil depends on a mean magnetic field orientation in the section of the radial cross-section of the magnet coil.

In a preferred embodiment, the mass distribution of the superconducting material over the radial cross-section of the magnet coil depends on or correlates with the distribution of the magnetic field strength and the vector field of the magnetic field over the radial cross-section of the magnet coil.

According to a further embodiment of the inventive magnet arrangement, the mass distribution of the superconducting material over the radial cross-section of the magnet coil depends on the vector field of the magnetic field over the radial cross-section of the magnet coil and an orientation of a crystalline structure of the superconducting material.

In taking into account the vector field of the magnetic field, but also the orientation of the crystalline structure of the superconducting material, the local distribution of the superconducting material may favourably be matched to local performance requirements of the superconducting material. Thus, costs and/or weight associated with the magnet arrangement may further be reduced in comparison to conventional magnet arrangements.

According to an embodiment of the inventive magnet arrangement, the magnet coil is subdivided into a plurality of axial segments. A mean width of the tape of a first axial segment differs from a mean width of the tape of a second axial segment of the plurality of axial segments.

The plurality of axial segments may represent imaginary or virtual axial segments of the magnet coil. For example, the magnet coil can be divided into an arbitrary number of imaginary axial segments or imaginary cylindrical coils with uniform or non-uniform dimensions.

As described above, the magnet coil may include one or more tapes comprising superconducting material. A mean width of a tape may represent an average width of the tape over an entire length of the tape. For example, the magnet coil may comprise a plurality of axially arranged tapes, each tape of the number of axially arranged tapes making exactly one turn or revolution around an axis of the magnet coil.

It is also conceivable, that one or more tapes of the plurality of tapes make a plurality of turns or revolutions around the axis of the magnet coil. In this case, the mean width of a tape may also represent an average width of a section of the tape, for example a section of the tape making a single revolution or a predefined number of revolutions around the axis of the magnet coil. The axis of the magnet coil may represent a cylinder axis or an axial of rotational symmetry of the magnet arrangement.

According to the invention, the mass distribution of the superconducting material over the radial cross-section of the magnet coil is dependent on a distribution of a magnetic field strength of the magnetic field over the radial cross-section of the magnet coil.

For example, a mean width of the first axial segment may exceed a mean width of the second axial segment. Preferably, the first axial segment is arranged closer to an axial end of the magnet coil compared to the second axial segment. The first axial segment may comprise a larger amount of superconducting material than the second axial segment. A larger amount of superconducting material may be required to sustain a higher magnet current which is needed to maintain the higher magnetic field strengths at the axial ends of the magnet coil. In an example, the first axial segment may have superconducting material at the axial end of the magnet coil where the local magnetic field strength is higher than over the second axial segment and will therefore require more superconducting material to carry the magnet current than does the second axial segment. In general, axial segments located closer to an axial end of the magnet coil may experience higher magnetic field strengths and therefore require more superconducting material to carry the current in comparison to axial segments located closer to an axial centre of the magnet coil.

In providing a magnet arrangement with at least a first axial segment and a second axial segment comprising different mean widths of tape, a mass distribution of superconducting material may favourably be adapted in consideration of the distribution of the magnetic field strength and/or the vector field of the magnetic field over the radial cross-section of the magnet coil.

In a preferred embodiment of the inventive magnet arrangement, a mean width of the tape decreases in a direction towards a geometric centre or a radial centre line of the radial cross-section of the magnet coil.

A geometric centre of the radial cross-section of the magnet coil may correspond to a centroid of a surface defined by the radial cross-section of the magnet coil. A radial centre line may correspond to a line which radially subdivides the radial cross-section of the magnet coil into two equally sized or symmetric segments.

For example, the widths of the one or more tapes of the plurality of axial segments may decrease in a direction towards the geometric centre of the radial cross-section of the magnet coil. It is also conceivable that one or more tapes of the plurality of axial segments exhibit a gradient in width along their lengths, particularly a gradient in width per one or more revolutions of the tape. Such a gradient in width may cause the mass of the superconducting material to decrease in the direction towards the geometric centre or the radial centre line of the radial cross-section of the magnet coil.

Different segments of the tape or different tapes of the magnet coil may exhibit different gradients in width. For example, the gradient in width of the tape may cause the mass of the superconducting material to increase in a direction towards an inner circumference (e. g. a radially inner surface) or an outer circumference (e. g. a radially outer surface) of the magnet coil. However, the gradient in width of the tape may also cause the mass of the superconducting material to decrease in the direction towards the geometric centre or the radial centre line of the radial cross-section of the magnet coil.

In an example, the magnet coil comprises a plurality of axially arranged cylindrical coils or segments, each comprising exactly one tape making one or more revolutions around the axis of the magnet coil. The widths of the tapes of the plurality of axially arranged cylindrical coils or segments may decrease in a direction towards the geometric centre or the radial centre line of the radial cross-section of the magnet coil.

In a further example, one or more tapes of the plurality of axially arranged cylindrical coils or segments exhibit a continuous or invariant width. In this case, the width of a cylindrical coil or segment located closer to the geometrical centre of the of the radial cross-section of the magnet coil may be lower than a width of a cylindrical coil or segment located further away from the geometrical centre of the of the radial cross-section of the magnet coil.

It is also conceivable, that a mean width per revolution of one or more tapes of the plurality of axially arranged cylindrical coils or segments decreases in the direction towards the geometric centre or the radial centre line of the radial cross-section of the magnet coil. Particularly, one or more tapes of each cylindrical coil or segment may exhibit a varying width or a gradient in width.

In providing a magnet coil comprising one or more tapes with varying widths, the mass distribution of the superconducting material may favourably be adjusted in dependence of the distribution of the magnetic field strength and/or the vector field of the magnetic field of the magnet arrangement in a cost-effective manner.

According to a further embodiment of the inventive magnet arrangement, the magnet coil is subdivided into a plurality of radial segments. A mean width of the tape of a first radial segment differs from a mean width of the tape of a second radial segment of the plurality of radial segments.

The plurality of radial segments may represent imaginary or virtual radial segments of the magnet coil. For example, the magnet coil can be subdivided into an arbitrary number of imaginary radial segments with uniform or non-uniform dimensions. However, the magnet coil may also be subdivided into a plurality of radially stacked tapes or windings of the tape. For example, a radial segment of the plurality of radial segments may correspond to a winding of a tape or a predefined number of windings of the tape. Thus, a radial segment may be defined by one or more revolutions or turns of the tape around the axis of the magnet coil.

As described above, the magnet coil may include one or more tapes comprising a superconducting material. A mean width of the tape may represent an average width of the tape over an entire length of the tape. For example, the magnet coil may comprise a plurality of radially stacked tapes, each tape of the plurality of radially stacked tapes making one or more turns or revolutions around an axis of the magnet arrangement. Preferably, each tape of the number of radially stacked tapes corresponds to a radial segment of the plurality of radial segments.

It is also conceivable that the magnet coil or an axial segment of the magnet comprises a single tape, making a plurality of turns or revolutions around the axis of the magnet coil. In this case, a radial segment may correspond to a single winding or a section of the tape making one full turn or revolution around the axis of the magnet coil.

The magnet coil may comprise at least a first radial segment and a second radial segment different from the first radial segment. The mean width of the tape of the first radial segment differs from the mean width of the of the tape of the second radial segment. Preferably, the first radial segment and the second radial segment are stacked in a radial direction of the magnet coil.

According to the invention, the mass distribution of the superconducting material over the radial cross-section of the magnet coil depends on the distribution of the magnetic field strength and/or the vector field of the magnetic field over the radial cross-section of the magnet coil.

For example, a mean width of the tape of the first radial segment exceeds a mean width of the tape of the second radial segment. Preferably, the first radial segment is arranged closer to a radially inner surface of the magnet coil compared to the second radial segment. The first radial segment may comprise a larger amount of superconducting material compared to the second radial segment. A larger amount of superconducting material may favourably allow for higher magnet currents to be sustained at the radially inner surface of the magnet coil.

A plurality of radial segments with different mean widths of the tape may favourably be stacked in a radial direction and thus, allow for the mass distribution of the superconducting material to be adjusted along the radial direction of the magnet coil.

According to a further embodiment of the inventive magnet arrangement, a mean width of the tape across the plurality of radial segments increases in a direction towards an inner circumference of the magnet coil.

For example, the magnet coil may comprise a plurality of stacked radial segments, each comprising exactly one tape making one turn or revolution around the axis of the magnet coil. In this case, the width of each tape may be constant or invariant over the length of each tape. However, radial segments located closer to the radially inner surface or inner circumference of the magnet coil may comprise a higher width in comparison to radial segments located closer to the geometric centre or the radial centre line of the of the radial cross-section of the magnet coil. Particularly, a mean width of the tape across the plurality of radial segments may decrease in a direction towards the geometric centre or the radial centre line of the radial cross-section of the magnet coil.

In a further example, the magnet coil may comprise a plurality of stacked radial segments, each comprising exactly one tape making an arbitrary amount of turns or revolutions around the axis of the magnet coil. The width of each tape of each radial segment may vary along the length of each tape. Particularly, a mean width per revolution of a radial segment located closer to the inner surface or inner circumference of the magnet coil may exceed a mean width per revolution of a radial segment located closer to the geometric centre or the radial centre line of the radial cross-section of the magnet coil.

In a preferred embodiment of the inventive magnet arrangement, the mean widths per revolution of the tapes of the plurality of radial segments decrease in a direction towards:
- the geometric centre or the radial centre line of the radial cross-section of the magnet coil,
- regions with lower magnetic field strength of the magnetic field and/or
- regions, where the magnetic vector field is oriented substantially in parallel to a crystalline structure of the superconducting material.

Particularly, the mean widths per revolution of the tapes of the plurality of radial segments may decrease in a direction towards regions, where the local field vector of the magnetic field is oriented substantially in parallel with an orientation of a crystalline structure of the superconducting material. For example, the mean widths per revolution of the tapes of the plurality of radial segments may decrease in a direction towards regions where a local performance of the tape is higher.

According to an embodiment of the inventive magnet arrangement, the magnet coil is subdivided into a plurality of axially arranged cylindrical coils or segments according to an embodiment described above. Each cylindrical coil or segment may be subdivided into one or more radial segments according to an embodiment described above.

By stacking radial segments or tapes with different mean widths in a radial direction or winding a tape with varying width around the axis of the magnet coil, the mass distribution of the superconducting material over the radial cross-section of the magnet coil may favourably be adjusted in consideration of a distribution of a magnetic field strength and/or a vector field of the magnetic field of the magnet coil, but also an orientation of the vector field with respect to an orientation of the crystalline structure of the superconducting material. Particularly, by stacking radial segments or tapes with different widths, or winding a tape with varying width around the axis of the magnet coil, a process of manufacturing a magnet coil of an inventive magnet arrangement may favourably be facilitated.

According to an embodiment of the inventive magnet arrangement, the magnet coil is axially subdivided into a plurality of cylindrical coils. A mean width of the tape of a first cylindrical coil differs from a mean width of the tape of a second cylindrical coil of the plurality of cylindrical coils.

As explained above, the magnet coil may be subdivided into a plurality of imaginary axial segments. However, the magnet coil may also be subdivided into a plurality of cylindrical coils, a plurality of "pancake coils", or a plurality of "double-pancake coils". For example, the magnet coil may be subdivided into a first cylindrical coil and a second cylindrical coil arranged concentrically along an axis, particularly an axis of rotational symmetry, defined by the magnet coil.

It is conceivable that the first cylindrical coil and the second cylindrical coil are electrically connected. For example, the first cylindrical coil may be electrically connected to the second cylindrical coil via a crossover turn. Particularly, the first cylindrical coil and the second cylindrical coil may form two coils of a double-pancake coil. Thus, only two current leads are required to electrically connect the first cylindrical coil and the second cylindrical coil to a power supply.

The magnet coil may comprise further cylindrical coils, e. g. a third cylindrical and a fourth cylindrical coil, arranged concentrically along the axis of the magnet coil.

It is conceivable that two adjacently arranged cylindrical coils form a double-pancake coil. The input leads of the cylindrical coils and/or double-pancake coils of the magnet coil may be electrically connected via a first electrical connector, e. g. a copper bar, a copper wire, a copper rail, a piece of superconducting material, or a similar structure comprising an electrically conducting material. Likewise, the output leads of the cylindrical coils and/or double-pancake coils may be electrically connected via a second electrical connector. The second electrical connector may be configured in accordance with the first electrical connector. Thus, the input leads and the output leads of the plurality of cylindrical coils and/or double-pancake coils may be electrically connected to a single power source via the first electrical connector and the second electrical connector.

In a preferred embodiment, the first cylindrical coil and the second cylindrical coil are wound using the "double pancake method". However, it is also conceivable that the first cylindrical coil and the second coil are individually connected to the external power supply.

According to an embodiment, the first cylindrical coil and the second cylindrical coil form a double-pancake coil according to an embodiment described above. The first cylindrical coil and the second cylindrical coil may be separated by an electrical insulator. Particularly, the electrical insulator may be co-wound with the tape when winding the double-pancake coil.

The first cylindrical coil and/or the second cylindrical coil may each comprise a single continuous tape or a plurality of tapes stacked in a radial direction of the magnet coil. It is conceivable that one or more tapes of the plurality of stacked tapes are electrically connected via a joint, for example a solder joint.

Preferably, the first cylindrical coil and the second cylindrical coil are arranged in such a way that the mass of superconducting material decreases in a direction towards the geometric centre of the radial cross-section of the magnet coil. For example, the mean width of the tape of the first cylindrical coil may exceed the mean width of the tape of the second cylindrical coil. As the magnetic field strength may be higher in proximity to the axial ends of the magnetic coil, the first cylindrical coil may be arranged closer to an axial end of the magnet coil in comparison to the second cylindrical coil. Preferably, the second cylindrical coil is located closer to a geometric centre of a radial cross-section of the magnet coil. Thus, a local mass distribution of superconducting material may be lower in regions of the magnet coil, which are exposed to lower magnetic field strengths.

In forming the magnet coil from a plurality of cylindrical coils, particularly a plurality of double-pancake coils, a manufacturing process of the inventive magnet arrangement may favourably be facilitated, while still allowing for the mass distribution of the superconducting material over the radial cross-section of the magnet coil to be adjusted as described above.

The inventive magnetic resonance device is configured to perform a magnetic resonance imaging examination of an object positioned within an imaging region of the magnetic resonance device. The magnetic resonance device comprises a magnet arrangement according to an embodiment described above.

Preferably, the magnetic resonance device is configured to acquire magnetic resonance image data, particularly diagnostic magnetic resonance image data, from the object positioned within the imaging region. The object may be an inanimate object or a patient, particularly a human or an animal.

The inventive magnetic resonance device may represent a closed bore scanner. A closed bore scanner may comprise a substantially cylindrical bore circumferentially enclosing the imaging region. The magnet arrangement of the closed bore scanner may comprise one or more cylindrical superconducting magnet coils according to an embodiment described above, circumferentially encompassing the imaging region along a section of a cylinder axis or an axis of rotational symmetry of the cylindrical bore. The one or more superconducting magnet coils may include a tape comprising a superconducting material according to an embodiment described above. The superconducting material may have a negligible electrical resistance at (or below) a superconducting temperature. A direction of a main magnetic field provided by the one or more cylindrical superconducting magnet coils may be oriented substantially in parallel to a direction of access of the object to the imaging region and/or the axial direction of the cylindrical bore.

The inventive magnetic resonance device may comprise further components required for a proper operation of the magnetic resonance device. For example, the magnetic resonance device may comprise an outer vacuum chamber, a magnet support structure, a thermal shield, a cryocooler, and the like. In certain embodiments, the magnetic resonance device comprises a cryogen vessel.

A cryogen vessel may be configured for storing or preserving a fluid, particularly a cryogen, at a predefined temperature level. Preferably, the fluid or cryogen exhibits a low boiling point. Examples of suitable fluids or cryogens are argon, nitrogen, neon, helium, hydrogen, or the like. The predefined temperature level may substantially correspond to a superconducting temperature of the superconducting material.

The cryocooler may represent a cooling device configured to cool the magnet coil of the magnet arrangement and/or maintain the magnet coil at a temperature level close to the superconducting temperature. The magnet arrangement, the thermal shield, the magnet support structure, and/or the cryogen vessel may be thermally connected to the cryocooler via a solid thermal conductor, a convection loop and/or a heat pipe.

The inventive magnetic resonance device may represent a "dry" system comprising a minimum of cryogen or no cryogen at all. For example, the inventive magnetic resonance device may comprise one or more small cryogen vessels thermally connected to the one or more magnet coils via a solid thermal conductor. The one or more small cryogen vessels may contain a volume of less than 10 l, less than 5 l, or less than 1 l of cryogen. According to an embodiment of the inventive magnetic resonance device, a cryogen vessel is omitted and the magnet arrangement is cooled entirely via thermal conduction.

In an alternative embodiment, the inventive magnetic resonance device represents a "wet" system. A "wet" system may include at least one cryogen vessel comprising a volume of more than 10 I or more than 100 I of cryogen. The magnet arrangement may be accommodated within the cryogen vessel and cooled directly by the cryogen. Particularly, a section of the magnet arrangement may be immersed in a liquid cryogenic fluid.

The magnetic resonance device shares the advantages of the inventive magnet arrangement according to an embodiment described above.

The inventive method of manufacturing a magnet coil of a magnet arrangement according to an embodiment described above comprises:
- cutting a raw tape in such a way to provide a tape comprising a lower width section and a higher width section along the length of the tape, and
- winding the tape into the magnet coil in such a way to provide a mass distribution of the superconducting material over the radial cross-section of the magnet coil which is dependent on a distribution of a magnetic field strength of the magnetic field over the radial cross-section of the magnet coil.

The cutting of the raw tape and the winding of the tape into the magnet coil may be carried out in such a way that the mass distribution of the superconducting material over the radial cross-section of the magnet coil is correlated with or depends on the distribution of the magnetic field strength of the magnetic field over the radial cross-section of the magnet coil. Particularly, a mass gradient of the superconducting material over the radial cross-section of the magnet coil may depend on a magnetic field gradient over the radial cross-section of the magnet coil.

In a preferred embodiment, the cutting of the raw tape and the winding of the tape into the magnet coil is carried out in such a way that the distribution of mass of the superconducting material over the radial cross-section of the magnet coil depends on the distribution of the magnetic field strength and the field vector of the magnetic field over the radial cross-section of the magnet coil. It is also conceivable that the cutting of the raw tape and the winding of the tape into the magnet coil is carried out in such a way that the mass of the superconducting material is distributed over the radial cross-section of the magnet coil in dependence of the distribution of the magnetic field orientation of the magnetic field relative to an orientation of a crystalline structure of the superconducting material.

The raw tape may represent a rectangular piece of tape comprising a thickness of about 0.1 to 0.2 mm and a width of 10 to 12 mm. However, the width and the thickness of the raw tape may deviate from the exemplary ranges dependent on the properties of the superconducting material, but also the intended application of the magnet arrangement.

In a preferred embodiment, the raw tape is cut in such a way to provide a smooth or continuous transition between the lower width section and the higher width section of the tape. For example, the width of the tape may be characterized by a linear or non-linear gradient. It is also conceivable, that the raw tape is cut in such a way to provide a tape comprising multiple sections with different gradients in width. In a further example, the raw tape is cut in such a way to provide a discontinuous or gradual transition between the lower width section and the higher width section of the tape.

The raw tape may be cut in such a way, that a length of the provided tape substantially corresponds to an inner circumference of the magnet coil, a multiple of the inner circumference of the magnet coil, or a non-integer multiple of the inner circumference of the magnet. For example, the raw tape may be cut in such a way to provide a tape configured to make exactly one turn or revolution around the axis defined by the magnet coil. In another example, the raw tape may be cut in such a way to provide a tape configured to make more than one turn or revolution around the axis defined by the magnet coil.

An inventive method may favourably allow for a magnet coil of a magnet arrangement according to an embodiment described above to be manufactured in an efficient, particularly a resource-efficient, manner. The inventive method shares the advantages associated with the inventive magnet arrangement.

According to an embodiment of the inventive method, the raw tape is cut in such a way that the lower width section of the tape is arranged at a region with lower magnetic field strength and the higher width section is arranged at a region with higher magnetic field strength when the tape is wound into the magnet coil of an inventive magnet arrangement.

The raw tape may be cut in such a way that a gradient in width and the length of tape are matched with a dimension, particularly an inner diameter and/or an inner circumference, of the magnet coil. Preferably, the tape is cut in such a way that a mean width of the tape per revolution decreases in a direction towards the geometric centre or the radial centre line of the radial cross-section of the magnet coil.

In an example, the tape is wound several times around the axis defined by the magnet coil. The tape may be cut in such a way that sections of the tape comprising higher widths are arranged in proximity to an inner circumference, but also an outer circumference, of the magnet coil, whereas sections of the tape comprising lower widths are arranged in proximity to a geometrical centre or a radial centre line of a radial cross-section of the magnet coil.

In order to increase the mass distribution of superconducting material at the inner circumference, and optionally the outer circumference, of the magnet coil, and decrease the mass distribution of superconducting material in proximity to the geometric centre or radial centre line of the radial cross-section of the magnet coil, the raw tape may be cut into an hour-glass shape, a trapezoidal shape, the shape of a concave pentagon, or the like. As described above, the tape may be subdivided into a plurality of radial segments or windings, and a mean width of the tape across the plurality of radial segments or windings may decrease in a direction towards the geometric centre or the radial centre line of the radial cross-section of the magnet coil.

In a preferred embodiment of the inventive method, the raw tape is cut in such a way that the lower width section of the tape is arranged at a region, where the magnetic field is oriented substantially in parallel with an orientation of the crystalline structure of the superconducting material. Particularly, the raw tape may be cut in such a way that the section with the lowest width of the tape is arranged at a region, where the local performance of the tape is highest.

In a preferred embodiment of the inventive method, the raw tape is cut in such a way, that a section of the tape comprises the shape of a hexagon, a concave pentagon, a trapezoid, or an hour-glass.

The raw tape may represent an initial state of the tape. Any properties of the raw tape may correspond to properties of the tape, except for one or more dimensions. The raw tape may comprise an elongated, substantially rectangular shape.

In a preferred embodiment of the inventive method, the raw tape is cut in such a way to minimize waste material.

For example, the rectangular raw tape may be cut into three sections along its length. A first section and a third section of the three sections may comprise the shape of a pentagon, and a second section of the three sections may comprise the shape of an hour-glass. The second section may be cut in half along its length to provide two further sections in the shape of concave pentagons. The first section and the third section may further be cut in half along a transversal direction to provide four trapezoidal shaped pieces of tape. It is conceivable that at least two pieces of tape cut from the raw tape are electrically connected via a joint.

In a further example, the rectangular raw tape is cut into three sections along its length. A first section and a third section of the three sections may comprise the shape of a concave pentagon, and a second section of the three sections may comprise the shape of a hexagon. The second section may be cut in half along a transversal direction to provide two sections in the shape of a trapezoid. It is conceivable that at least two pieces of cut tape are electrically connected via a joint.

It is also conceivable that the raw tape is cut into sections comprising the shape of a triangle, a rhombus, or the like. In this case, tips at the longitudinal ends of the cut tape may be cut off to provide a desired minimum width of the tape.

In a preferred embodiment, the raw tape is cut in such a way that less than 20 %, less than 15 %, less than 10 %, or less than 5 % of the superconducting material is discarded.

The inventive method may favourably allow for a resource-efficient use of the superconducting material. Thus, manufacturing costs may favourably be reduced in comparison to conventional methods of manufacturing superconducting magnets.

According to an embodiment, the inventive method comprises:
- cutting a plurality of separate raw tapes in such a way to provide a plurality of tapes, each tape of the plurality of tapes comprising a lower width section and a higher width section,
- winding each tape of the plurality of tapes into a cylindrical coil to provide a plurality of cylindrical coils, and
- forming the magnet coil by axially aligning the plurality of cylindrical coils.

Each tape of the plurality of separate raw tapes may be cut according to an embodiment described above. It is conceivable that each cylindrical coil of the plurality of cylindrical coils comprises exactly one tape. However, it is also conceivable that one or more cylindrical coils of the plurality of cylindrical coils comprise a plurality of tapes. For example, the tapes may be stacked in a radial direction and/or form radial segments of the cylindrical coil according to an embodiment described above. In case a cylindrical coil comprises a plurality of tapes, the superconducting material of the plurality of tapes may be electrically connected via a superconducting joint.

Winding the one or more tapes into a cylindrical coil may comprise winding the one or more cylindrical coils onto a former, a magnet support structure, or an arbitrary cylindrical substrate.

The magnet may be formed by axially aligning or arranging the plurality of cylindrical coils. Preferably, the plurality of cylindrical coils is arranged coaxially along the axis defined by the magnet coil. It is conceivable that one or more cylindrical coils of the plurality of cylindrical coils are arranged directly adjacent to one another. However, the one or more cylindrical coils of the plurality of cylindrical coils may also be separated from one another by a spacer and/or an electrically insulating material. Preferably, the one or more cylindrical coils of the plurality of cylindrical coils are integrally bonded according to an embodiment described above.

One or more cylindrical coils of the plurality of cylindrical coils may be electrically connected via a joint. For example, two cylindrical coils of the plurality of cylindrical coils may be electrically connected via a crossover turn. Particularly, two adjacent cylindrical coils of the plurality of cylindrical coils may form a double-pancake coil according to an embodiment described above.

According to an embodiment, the winding of each tape of the plurality of tapes into a cylindrical coil may comprise winding at least two tapes of the plurality of tapes into a double-pancake coil using the double-pancake method.

The inventive magnet arrangement may comprise a plurality of magnet coils. Each magnet coil of the plurality of magnet coils may be manufactured according to an embodiment of the inventive method described above.

The inventive method may favourably allow for tuning a radial mass distribution of the superconducting material in the plurality of cylindrical coils by winding one or more tapes with varying width into cylindrical coils. Particularly, the radial mass distribution of the superconducting material within the magnet coil may be adjusted via the gradient in width (e. g. dependent on the shape of the cut tape) and the number of turns of the one or more tapes.

An axial mass distribution of the superconducting material may favourably be adjusted via the mean widths, but also the gradient in width, of the one or more tapes in each cylindrical coil of the plurality of coils forming the magnet coil.

The inventive method my favourably provide a particularly simple and/or cost-effective process for manufacturing an inventive magnet arrangement.

According to a further embodiment, the inventive method comprises:
- co-winding a filler material with the tape in such a way that the filler material fills in gaps created by sections of the tape with a smaller width.

Preferably, the filler material is mechanically and/or thermally matched with the materials of the tape (e. g. the substrate and/or the superconducting material) in order to reduce a risk of cracking of the superconducting material due to different thermal expansion coefficients. For example, the filler material may comprise or consist of an electrically conducting metal, such as copper, silver, or aluminium, or a stainless-steel alloy according to an embodiment described above.

The filler material may be co-wound with the tape in such a way to compensate for a varying width of the tape. Particularly, the filler material may be co-wound with the tape in such a way to provide a magnet coil or a cylindrical coil with uniform width in a radial cross-section.

Co-winding a filler material with the tape may favourably allow for a provision of a magnet coil or a cylindrical coil, which has a constant or uniform width along a radial direction. Thus, a process of assembling one or more magnet coils into an inventive magnet assembly or forming an inventive magnet coil from a plurality of cylindrical coils may favourably be facilitated. Furthermore, a filler material co-wound with the tape may favourably increase a mechanical stability of the inventive magnet arrangement.

In a preferred embodiment of the inventive method, the cutting of the raw tape is conducted using a LASER cutting process.

The raw tape may be cut, for example, using a mechanical cutting process or a LASER cutting process. LASER cutting may involve directing a high-intensity LASER beam onto a surface of the raw tape. The LASER beam may locally ablate through the superconducting material and the substrate, thus cutting the raw tape. In using a LASER cutting technique, mechanical forces on the tape may favourably be reduced or avoided. However, an operation of LASER equipment is typically associated with higher costs in comparison to mechanical cutting equipment.

Alternatively, a mechanical cutting process may be used to cut the raw tape. However, mechanical cutting processes are less preferred due to the risk of micro-cracks forming in the superconducting material and/or the substrate by the mechanical forces on the tape. These micro-cracks may act as initiation sites for continued crack formation and propagation.

An inventive method according to an embodiment described above may favourably allow for an efficient use of superconducting material. Thus, costs and/or a weight of the inventive magnet arrangement may be reduced in comparison to conventional superconducting magnets.

Further advantages and details of the present invention may be recognized from the embodiments described below as well as the drawings. The figures show:
- Fig. 1: a schematic representation of an embodiment of an inventive magnetic resonance device,
- Fig. 2: an example of a distribution of a magnetic field strength of a magnetic field over a radial cross-section of a magnet coil,
- Fig. 3: an example of a tape performance in dependence of a distribution of a magnetic field strength and magnetic field orientation of a magnetic field over a radial cross-section of a magnet coil,
- Fig. 4: a schematic representation of a magnet coil of an inventive magnet arrangement,
- Fig. 5: a schematic representation of a magnet coil of an inventive magnet arrangement,
- Fig. 6: a schematic representation of a magnet coil of an inventive magnet arrangement,
- Fig. 7: a schematic representation of a conventional process of cutting raw tape,
- Fig. 8a: a schematic representation of a process of cutting raw tape according to an inventive method,
- Fig. 8b: a schematic representation of a process of cutting raw tape according to an inventive method,
- Fig. 9a: a schematic representation of a process of cutting raw tape according to an inventive method,
- Fig. 9b: a schematic representation of a process of cutting raw tape according to an inventive method,
- Fig. 10: a schematic representation of an embodiment of an inventive magnet arrangement,
- Fig. 11: a schematic representation of a flow chart of a method according to the invention,
- Fig. 12: a schematic representation of a flow chart of a method according to the invention.

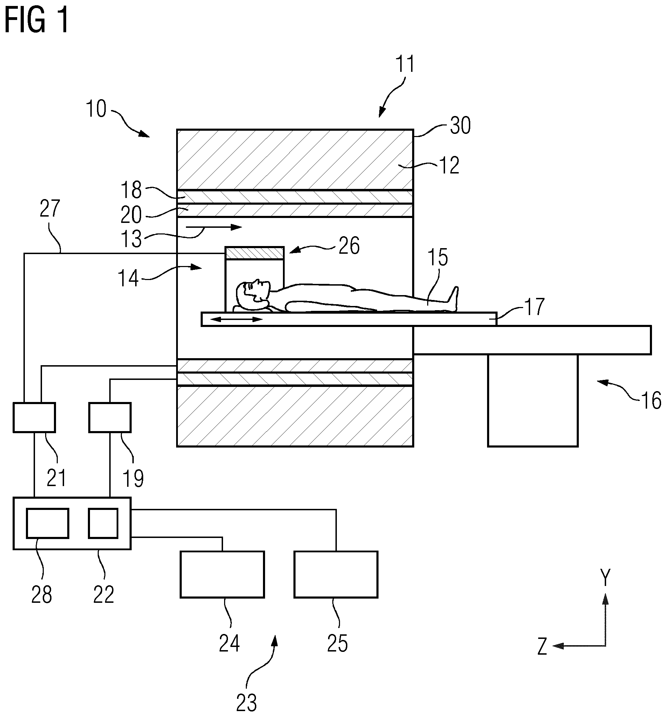

Fig. 1 shows an embodiment of a magnetic resonance device 10 according to the invention. The magnetic resonance device 10 comprises a static field magnet or main magnet 12 configured to provide a homogenous, static magnetic field 13 (B0 field). The static magnetic field 13 permeates an imaging region 14 configured to receive an imaging object, such as a patient 15. The imaging region 14 may correspond to a patient bore configured to accommodate a patient 15 during a magnetic resonance measurement. The imaging region 14 is encompassed by the main magnet 12 in a circumferential direction. The main magnet 12 forms a part of an inventive magnet arrangement 11.

The magnetic resonance device 10 may comprise a patient positioning device 16 configured to transport the patient 15 into the imaging region 14. Particularly, the patient positioning device 16 may be configured to transport a diagnostically relevant body region of the patient 15 into an imaging volume (not shown) of the magnetic resonance device 10. The magnet arrangement 11 and other components of the magnetic resonance device 10 may be concealed in a housing 30.

The magnetic resonance device 10 may comprise a gradient system including one or more gradient coils 18. The one or more gradient coils 18 may be configured to generate gradient magnetic fields in different, preferably orthogonally oriented, spatial directions. The gradient magnetic fields can be used for spatial encoding of magnetic resonance signals or magnetic resonance data acquired during a magnetic resonance measurement. The one or more gradient coils 18 can be activated or controlled via an appropriate control signal provided by a gradient control unit 19.

The magnetic resonance device 10 may further comprise an integrated radiofrequency antenna 20 (i. e. a body coil). The radiofrequency antenna 20 may be activated or controlled via a radiofrequency control unit 21. The radiofrequency control unit 21 may be configured to control the radiofrequency antenna 20 to generate a high frequency magnetic field and emit radiofrequency excitation pulses into the imaging region 14. The magnetic resonance device 10 may further comprise a local coil 26. The local coil 26 may be positioned on or in proximity to the diagnostically relevant body region of the patient 15. The local coil 26 may be configured to emit radiofrequency excitation pulses into the patient 15 and/or receive magnetic resonance signals from the patient 15. It is conceivable, that the local coil 26 is controlled via the radiofrequency controller 21.

Preferably, the magnetic resonance device 10 comprises a control unit 22 configured to control the magnetic resonance device 10. The control unit 22 may comprise a processing unit 28 configured to process magnetic resonance signals and reconstruct magnetic resonance images. The processing unit 28 may also be configured to process input from a user of the magnetic resonance device 10 and/or provide an output to a user. For this purpose, the processing unit 28 and/or the control unit 22 can be connected to a display unit 24 and an input unit 25 via a suitable signal connection. For a preparation of a magnetic resonance measurement, preparatory information, such as imaging parameters or patient information, can be provided to the user via the display unit 24. The input unit 25 may be configured to receive information and/or imaging parameters from the user.

Of course, the magnetic resonance device 10 may comprise further components and/or functions which are common in magnetic resonance devices. The general operation of a magnetic resonance device 10 is known to those skilled in the art, so a more detailed description is omitted.

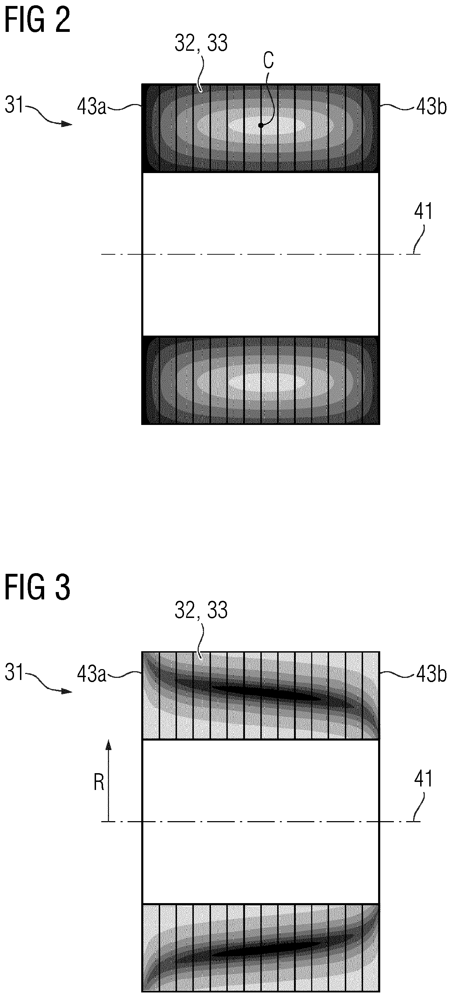

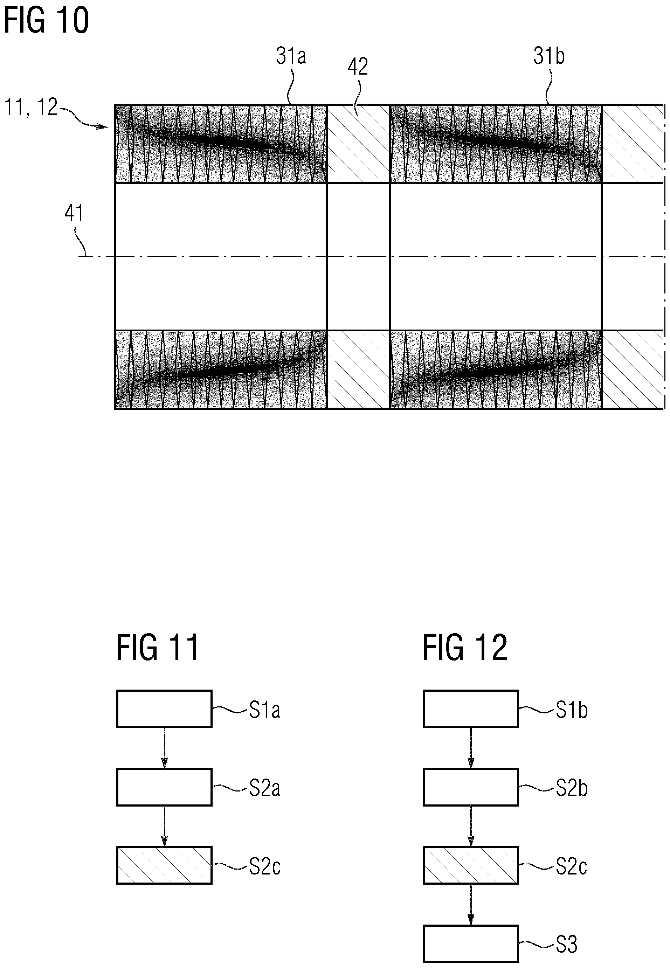

Fig. 2 shows an embodiment of a magnet coil 31 of a magnet arrangement 11. The magnet coil 31 includes a tape 32 comprising a superconducting material wound around an axis 41 of the magnet coil 31. The magnet coil 31 may represent a cylindrical coil and the axis 41 may correspond to a cylinder axis or an axis of rotational symmetry of the magnet coil 31 and/or the magnet arrangement 11 (see Fig. 10).

Preferably, the superconducting material comprises or consists of a high temperature superconductor. The tape 32 may comprise a substrate carrying and/or supporting the superconducting material. The substrate may comprise or consist of a stainless-steel alloy according to an embodiment described above.

Fig. 2 provides a schematic representation of a distribution of a magnetic field strength of a magnetic field over a radial cross-section of a magnet coil 31. During an operation of a magnetic resonance device 10 comprising an inventive magnet arrangement 11, higher magnetic field strengths may occur near the axial ends 43a and 43b of the magnet coil 31, whilst a lower magnetic field strength may occur near the geometric centre C of the radial cross-section of the magnet coil 31.

According to the present invention, the mass of superconducting material may be reduced in regions with lower magnetic field strength and increased in regions with higher magnetic field strength by adjusting a width .

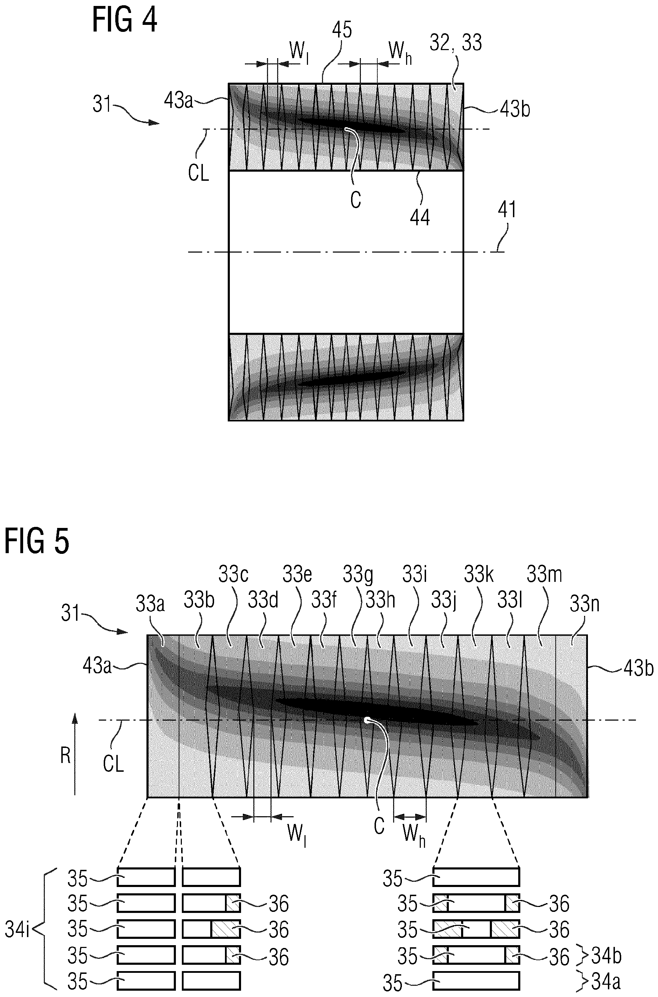

Fig. 3 shows a schematic representation of a tape performance in dependence of the distributions of the magnetic field strength and the magnetic field orientation of the magnetic field over radial cross-sections of a magnet coil 31. For example, the brighter areas of the radial cross-section of the magnet coil 31 may designate regions where a performance of the superconducting material of the tape 32 is lower, thus impeding a flow of current through the superconducting material. In contrast, the darker areas of the radial cross-section of the magnet coil 31 may designate regions where the performance superconducting material of the tape is higher, thus facilitating a flow of current through the superconducting material. The tape performance encoded in the grey values (or brightness) may depend on the distribution of the magnetic field strength of the magnetic field (see Fig. 2), but also the distribution of the magnetic field orientation (i. e. the vector field) of the magnetic field as well as an orientation of the crystalline structure of the superconducting material. For example, the darker areas of the radial cross-section of the magnet coil 31 may designate regions where the local field vector of the magnetic field is oriented substantially in parallel to the orientation of the crystalline structure of the superconducting material. The brighter areas, of the radial cross-section of the magnet coil 31 may designate regions where the local field vector of the magnetic field is oriented substantially perpendicular to the orientation of the crystalline structure of the superconducting material. It is conceivable that the darker areas represent regions of lower magnetic field strength, and the brighter areas represent regions of higher magnetic field strength. According to the invention, narrower tape may be used in the darker areas, and wider tape may be used in the brighter or lighter areas to efficiently match the distribution of superconducting material to the background magnetic field (see Fig. 4).

The local performance of the superconducting material may depend on an angle that the background magnetic field makes with a layer of superconducting material on the tape 32. For example, the performance of the tape 32 may be highest when the field vector of the background magnetic field is oriented perpendicular to the orientation of the crystalline structure of the superconducting material and lowest when the field vector of the background magnetic field is oriented in parallel with the orientation of the crystalline structure of the superconducting material. Thus, the tape performance may vary in dependence of the local orientation of the field vector of the background magnetic field.

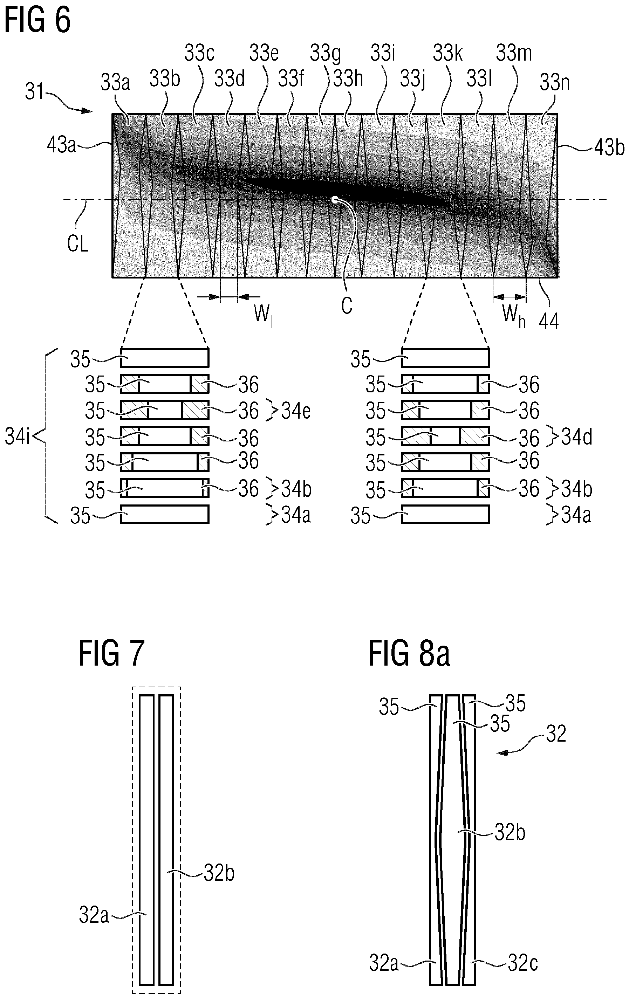

Similar to the embodiment depicted in Fig. 2, the magnet coil 31 in Fig. 3 comprises a tape 32 which is wound around the axis 41 of the magnet coil 31. The tape 32 may be wound into a plurality of cylindrical coils which are concentrically arranged along the axis 41. It is conceivable that the tape 32 is wound in such a way to provide stacked layers of tape 32 in a radial direction R of the magnet coil 31 (see Figures 5 and 6).

The Figures 2 and 3 show schematically how the magnet coil 31 may be subdivided into a plurality of imaginary or physically separated cylindrical coils 33 or double pancake coils. Each cylindrical coil 33 or double pancake coil may comprise one or more tapes 32 with varying width (not shown in Fig. 2 and 3). The variations in tape width may be compensated using a filler material 36 (see Figures 5 and 6) to increase mechanical stability of the magnet coil 31.

In a preferred embodiment, the mass distribution of the superconducting material varies over the radial cross-section of the magnet coil 31 in dependence of the distribution of the magnetic field strength, but also the local orientation, of the magnetic field.

Fig. 4 shows an embodiment of a magnet coil 31 of an inventive magnet arrangement 11. In the depicted example, the width of the tape 32 varies over the length of the tape 32 in such a way, that the mass distribution of the superconducting material over the radial cross-section of the magnet coil 31 depends on the distributions of the magnetic field strength and the magnetic field orientation of the magnetic field over the radial cross-section of the magnet coil 31.The mass distribution of the superconducting material over the radial cross-section of the magnet coil 31 may also depend on the orientation of the crystalline structure of the superconducting material as described above.

In the depicted example, the widths Wₗ of the turns of the tape 32 arranged in proximity to a geometric centre C or a radial centre line CL of the radial cross-section of the magnet coil 31 are generally lower in comparison to the widths Wₕ of the turns of the tape 32 located closer to an inner circumference 44 or an outer circumference 45 of the magnet coil 31. Thus, a mean width per revolution of the tape 32 may decrease in a direction towards the geometric centre C or radial centre line CL of the radial cross-section of the magnet coil 31. The mean widths of the tapes 32 of the cylindrical coils 33 may increase in a direction towards an axial end 43a, 43b of the magnet coil 31.

In a preferred embodiment, the mean widths per revolution of the tapes 32 decrease in a direction towards the regions with highest performance of the tapes 32 (i. e. the darkest regions in Fig. 4) of the magnet coil 31.

Fig. 5 shows an embodiment of a magnet coil 31 of an inventive magnet arrangement 11. In the depicted example, the magnet coil 31 comprises a plurality of cylindrical coils 33a-n or double pancake coils, which are arranged concentrically along the axis 41 (not shown) of the magnet coil 31. Each cylindrical coil 33a-n may comprise or consist of one or more tapes 32. Preferably, each cylindrical coil 33 comprises a plurality of windings or layers 34i of the tape 32. The layers 34i of tape 32 may be stacked in a radial direction R of each cylindrical coil 33.

In the embodiment depicted in Fig. 5, the mean width per revolution of the tape 32 of the cylindrical coil 33a is constant across all layers 34i of the tape 32. In contrast, the mean width per revolution of the tape 32 of the cylindrical coil 33k decreases towards the geometrical centre C or radial centre line CL of the radial cross-section of the magnet coil 31. Thus, the mean width of the tape 32 of the cylindrical coil 33a exceeds the mean width of the tape 32 of the cylindrical coil 33k. It is conceivable that the mean widths of the tapes 32 of the cylindrical coils 33a-n generally increase in a direction from the geometric centre C towards the axial ends 43a and 43b of the magnet coil 31.

Preferably, the mean widths per revolution of the tape 32 of the cylindrical coil 33k, but also other cylindrical coils 33b-m, decreases towards the geometric centre C or the radial centre line CL of the radial cross-section of the magnet coil 31. Particularly, the mean widths per revolution of the tapes 32 of the coils 33b-m may decrease towards the region of highest tape performance (e. g. the darkest regions) as described above.

In the embodiment depicted in Fig. 5, the magnet coil 31, but also each cylindrical coil 33a-n, is subdivided into a plurality of radial segments which may correspond to the layers 34i of the tape 32 (the substrate carrying the superconducting material 35 is not shown). Referring to the example of the cylindrical coil 33k, a mean width of the tape 32 of a first radial segment 34a differs from a mean width of the tape 32 of a second radial segment 34b. The mean widths per revolution of the tape 32 may decrease towards the geometrical centre C or the radial centre line CL of the radial cross-section of the magnet coil 31 as described above. Particularly, the mean width of each layer 34i may depend on or correlate with the local tape performance, which in turn may depend on the distributions of the magnet field strength and the magnetic field orientation of the magnetic field generated via the magnet arrangement 11, but also the spatial orientation of the crystalline structure of the superconducting material 35.

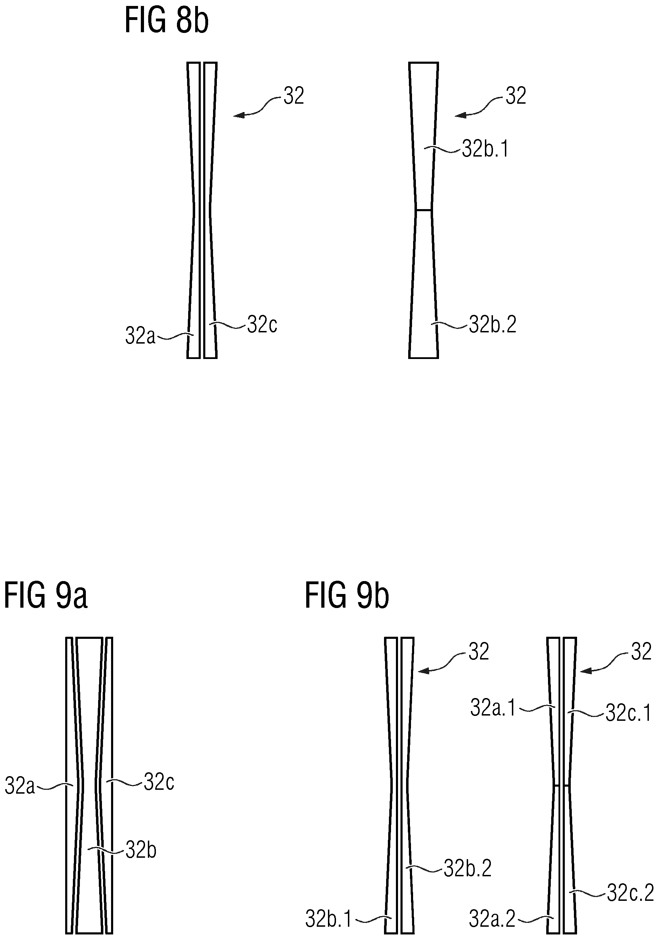

Each cylindrical coil 33 may include one or more tapes 32 comprising a superconducting material 35 and a filler material 36 which is co-wound with the tape 32 as depicted in Fig. 5. As the width of the tape 32 of the cylindrical coil 33a is constant, a filler material 36 is co-wound only on a side of the cylindrical coil 33b facing away from the cylindrical coil 33a. For example, the tape 32 used in the cylindrical coil 33b may represent a section 32a or 32c left over from cutting the section 32b of the cylindrical coil 33c from a rectangular raw tape (see Figures 8a and 8b), thus allowing for a resource-efficient use of superconducting material.

Fig. 6 shows a further embodiment of a magnet coil 31 of an inventive magnet arrangement 11, which is similar to the example depicted in Fig. 5. In contrast to the previous example, the mean width per revolution of the tape 32 of the cylindrical coil 33a varies across the length of the tape 32.

In the present example, the one or more tapes 32 of each cylindrical coil 33a-n are co-wound with a filler material 36. The filler material 36 compensates for the varying width of the layers or windings 34i of the tapes 32 on both axial ends of each cylindrical coil 33a-n.

It is conceivable that a distance between a winding or layer with the lowest width and the inner circumference 44 of the magnet coil 31 differs across all cylindrical coils 33a-n. In Fig. 6, the layer 34e represents the winding or layer with the lowest width of the cylindrical coil 33b, whereas the layer 34d represents the winding or layer with the lowest width of the cylindrical coil 33h. The minimum distance between the layer 34e of cylindrical coil 33b and the inner circumference 44 of the magnet coil 31 exceeds the minimum distance between the layer 34d of cylindrical coil 33h and the inner circumference 44 of the magnet coil 31. The mean width per revolution of the tapes 32 of the cylindrical coils 33a-n decreases towards the darkest regions of the radial cross-section of the magnet coil 31, where the local tape performance (i. e. the performance of the superconducting material) is highest.

Fig. 7 depicts a conventional method of cutting a raw tape 32 for use in a superconducting magnet coil. The raw tape comprises a rectangular shape which is cut along the length to provide two rectangular strips or tapes 32a and 32b. The two strips or tapes 32a and 32b may be wound into one or more cylindrical coils and formed into a magnet coil. Magnet coils formed from rectangular tapes 32a and 32b exhibit a substantially constant mass distribution of superconducting material over the radial cross-section of the magnet coil 31 and do not provide the benefits associated with the magnet coil 31 in an inventive magnet arrangement 11.

Fig. 8a depicts tapes 32 cut in accordance with an inventive method of manufacturing a magnet arrangement 11. The rectangular raw tape is cut along its length into three sections 32a, 32b and 32c. The outer sections 32a and 32c may comprise the shape of a concave pentagon, whereas the middle section 32b may comprise the shape of a hexagon.

Fig. 8b shows how the sections 32a, 32b, and 32c (32a-c) can be assembled or arranged to provide tapes 32 with varying widths for use in a magnet coil 31 of an inventive magnet arrangement 11 according to an embodiment described herein.

For example, the middle section 32b can be cut transversally at the section of highest width to provide two sections 32b.1 and 32b.2 of trapezoidal shape. The two edges with the lowest width may then be connected via a superconducting joint to provide a tape comprising the shape of an hour-glass.

The outer sections 32a and 32c may be used in a cylindrical coil 33b, for example in conjunction with a single rectangular tape 32 (see Fig. 5). However, the outer sections 32a and 32c may also be arranged in such a way that the straight sides face each other to provide an hour-glass shape which can be used in a cylindrical coil 33k (see Figures 5 and 6). It is conceivable that the outer sections 32a and 32c are electrically connected to each other and/or to another tape 32 via a superconducting joint.

Fig. 9 depicts a further example of a raw tape cut in accordance with an inventive method of manufacturing a magnet arrangement 11. The rectangular raw tape is cut into three sections 32a, 32b and 32c along its length. The first section 32a and the third section 32c comprise the shape of a pentagon, whereas the second section 32b comprises the shape of an hour-glass.

Fig. 9b shows how the sections 32a, 32b, and 32c (32a-c) can be assembled or arranged to provide tapes 32 with varying widths for use in a magnet coil 31 of an inventive magnet arrangement 11 according to an embodiment described herein.

For example, the second section 32b may be cut in half along its length to provide two further sections 32b.1 and 32b.2 in the shape of concave pentagons. The two sections 32b.1 and 32b.2 may be rearranged and used in accordance with the sections 32a and 32b shown in Fig. 8b.

The first section 32a and the third section 32b may further be cut in half along a transversal direction to provide four trapezoidal shaped sections 32a.1, 32a.2, 32c.1 and 32c.2. It is conceivable that the two sections 32a.1 and 32a.2, but also the two sections 32c.1 and 32c.2, are electrically connected via a superconducting joint to provide a tape 32 comprising the shape of a concave pentagon (or a half of an hour-glass). Two concave pentagons may be electrically connected to form an hour-glass shape or wound into a cylindrical coil 33b as shown in Fig. 5.

Fig. 10 shows an embodiment of an inventive magnet arrangement 11. The magnet arrangement 11 may comprise a plurality of magnet coils 31 which form the main magnet 12 of an inventive magnetic resonance device 10 according to Fig. 1.

In the depicted example, the magnet arrangement 11 comprises at least two magnet coils 31a and 31b arranged concentrically along the cylinder axis 41 or axis of rotational symmetry of the magnet arrangement 11. The two magnet coils 31a and 31b are divided via a spacer 42. The magnet coil 31a and the magnet coil 31b may be attached to the spacer 42 via a force-locking connection, a form-locking connection and/or a material bond. Preferably, the magnet coil 31a, the magnet coil 31b and the spacer 42 are integrally bonded to form a coherent or cohesive structure.

Preferably, the spacer 42 is implemented as a ring, a hollow cylinder, or a plurality of segments of a ring or a hollow cylinder. It is also conceivable that the spacer 42 comprises one or more blocks of any suitable shape.

Of course, the magnet arrangement 11 may comprise further magnet coils 31 and/or spacers 42.

Fig. 11 depicts a schematic flow chart of an embodiment of an inventive method of manufacturing an inventive magnet arrangement 11.

In a step S1a, the raw tape is cut in such a way to provide a tape 32 comprising a lower width section and a higher width section along the length of the tape 32.

For example, a rectangular raw tape can be cut into three sections 32a, 32b and 32c as shown in Figures 8a and 9a.

In a preferred embodiment, the raw tape is cut in such a way to provide a smooth or continuous transition between the lower width section and the higher width section. For example, a variation of the width of the tape 32 along the length of the tape 32 may be defined by a linear or non-linear gradient. It is also conceivable, that the raw tape is cut in such a way that multiple sections with different gradients in width are provided.

According to an embodiment, the raw tape is cut in such a way that the transition between the lower width section and the higher width section is discontinuous or gradual.

The raw tape may be cut in such a way, that a length of the tape 32 substantially corresponds to an inner circumference of the magnet coil 31, a multiple of the inner circumference of the magnet coil 31, or a non-integer multiple of the inner circumference of the magnet 31. For example, the tape 32 may be cut in such a way that it makes exactly one turn or revolution around the axis 41 (see Fig. 4) defined by the magnet coil 31. In another example, the tape 32 may be cut in such a way that it makes more than one turn or revolution around the axis 41 defined by the magnet coil 31.

According to an embodiment, the step S1a comprises cutting the raw tape in such a way that the lower width section of the tape 32 can be arranged at a high-performance region and the higher width section can be arranged at a low-performance section of the magnet coil 31 when the tape is wound into the magnet coil (see Fig. 4).

Preferably, the step S1a comprises cutting the raw tape in such a way that a section of the tape comprises the shape of a hexagon, a concave pentagon, a trapezoid, or an hourglass. For example, the raw tape may be cut into two or more sections, e. g. section 32a, 32b and 32c as shown in Figures 8a or 9a. It is also conceivable that sections of the tape 32 are subsequently cut into further sections 32b.1 and 32b.2 as shown in Fig. 8b, or further sections 32b.1, 32b.2, 31a.1, 31a.2, 31c.1 and 31c.2 as shown in Fig. 9b.

According to a less preferred embodiment, the raw tape is cut into the shape of a rhombus or a triangle. In this case, tips of the tape 32 may be cut off in a subsequent step to provide the sections of lower width.

In a step S2a, the tape 32 is wound into the magnet coil 31 in such a way to provide a mass distribution of the superconducting material over the radial cross-section of the magnet coil which is dependent on a distribution of a magnetic field strength of the magnetic field over the radial cross-section of the magnet coil.

The tape 32 may be wound into the magnet coil 31 using a former or a cylindrical substrate according to an embodiment described above. Particularly, the tape 32 may be wound into cylindrical coils 33, which may be axially arranged to form the magnet coil 31. Preferably, the tape 32 is wound into a plurality of double-pancake coils using the double-pancake method.

According to a preferred embodiment, the inventive method comprises:
- cutting S1b a plurality of separate raw tapes in such a way to provide a plurality of tapes 32, each tape 32 of the plurality of tapes 32 comprising a lower width section and a higher width section,
- winding S2b each tape 32 of the plurality of tapes 32 into a cylindrical coil 33 to provide a plurality of cylindrical coils 33, and
- forming S3 the magnet coil 31 by axially aligning the plurality of cylindrical coils 31.

The step S3 of forming the magnet coil 31 may comprise concentrically arranging the plurality of cylindrical coils 31 along the axis 41 of the magnetic coil 31. Preferably, pairs of cylindrical coils 33 are wound as double-pancake coils which are concentrically arranged along the axis 41 to form the magnet coil 31.

According to an embodiment, the inventive method comprises the optional step S2c of co-winding a filler material 36 with the tape 32 in such a way, that the filler material fills in gaps created by the sections of the tape 32 with lower width.

The embodiments described herein are to be recognized as examples. It is to be understood that individual embodiments may be extended by or combined with features of other embodiments if not explicitly stated otherwise. Particularly, the sequence of the steps of the inventive method is to be understood as an example. Individual steps may be carried out in a different order and/or overlap partially or completely in time. The embodiments depicted in the Figures are representations that do not necessarily have to be to scale.

## Claims

1. Magnet arrangement (11) for a magnetic resonance device (10), configured to provide a magnetic field suitable for use in a magnetic resonance imaging examination, including a magnet coil (31) wound from a tape (32) comprising a superconducting material (35),
wherein a width of the tape (32) varies along on a length of the tape (32), and
wherein a mass distribution of the superconducting material (35) over a radial cross-section of the magnet coil (31) is dependent on a distribution of a magnetic field strength of the magnetic field over the radial cross-section of the magnet coil (31).

2. Magnet arrangement (11) according to the claim 1, wherein the mass distribution of the superconducting material (35) over the radial cross-section of the magnet coil (31) is dependent on a vector field of the magnetic field over the radial cross-section of the magnet coil (31).

3. Magnet arrangement (11) according to one of the claims 1 or 2, wherein the superconducting material (35) is a high temperature superconducting material.

4. Magnet arrangement (11) according to one of the preceding claims, wherein the magnet coil (31) is subdivided into a plurality of axial segments, and wherein a mean width of the tape (32) of a first axial segment differs from a mean width of the tape (32) of a second axial segment of the plurality of axial segments.

5. Magnet arrangement (11) according to one of the preceding claims, wherein a mean width of the tape (32) decreases in a direction towards a geometric centre (C) or a radial centre line (CL) of the radial cross-section of the magnet coil (31).

6. Magnet arrangement (11) according to one of the preceding claims, wherein the magnet coil (31) is subdivided into a plurality of radial segments, and wherein a mean width of the tape (32) of a first radial segment differs from a mean width of the tape (32) of a second radial segment of the plurality of radial segments.

7. Magnet arrangement (11) according to claim 6, wherein a mean width of the tape (32) across the plurality of radial segments increases in a direction towards an inner circumference of the magnet coil (31).

8. Magnet arrangement (11) according to one of the preceding claims, wherein the magnet coil (31) is axially subdivided into a plurality of cylindrical coils (33), and wherein a mean width of the tape (32) of a first cylindrical coil (33) differs from a mean width of the tape (32) of a second cylindrical coil (33) of the plurality of cylindrical coils (33).

9. Magnetic resonance device (10), configured to perform a magnetic resonance imaging examination of an object positioned within an imaging region of the magnetic resonance device (10), comprising a magnet arrangement (11) according to one of the preceding claims.

10. Method of manufacturing a magnet coil (31) of a magnet arrangement (11) according to one of the preceding claims 1 to 8, comprising:
• cutting (S1a) a raw tape in such a way to provide a tape (32) comprising a lower width section and a higher width section along the length of the tape (32), and
• winding (S2a) the tape (32) into the magnet coil (31) in such a way to provide a mass distribution of the superconducting material (35) over the radial cross-section of the magnet coil (31) which is dependent on a distribution of a magnetic field strength of the magnetic field over the radial cross-section of the magnet coil (31).

11. The method according to claim 10, wherein the raw tape is cut (S1a) in such a way that the lower width section of the tape (32) is arranged at a region with lower magnetic field strength and the higher width section is arranged at a region with higher magnetic field strength when the tape (32) is wound into the magnet coil (31) of an inventive magnet arrangement (11).

12. The method according to one of the claims 10 or 11, wherein the raw tape is cut (S1a) in such a way, that a section of the tape (32) comprises the shape of a hexagon, a concave pentagon, a trapezoid, or an hourglass.

13. The method according to one of the preceding claims 10 to 12, comprising:
• cutting (S1b) a plurality of separate raw tapes in such a way to provide a plurality of tapes (32), each tape (32) of the plurality of tapes (32) comprising a lower width section and a higher width section,
• winding (S2b) each tape (32) of the plurality of tapes (32) into a cylindrical coil (33) to provide a plurality of cylindrical coils (33), and
• forming (S3) the magnet coil (31) by axially aligning the plurality of cylindrical coils (33).

14. The method according to one of the claims 10 to 13, further comprising:
• co-winding (S2c) a filler material (36) with the tape (32) in such a way that the filler material (36) fills in gaps created by sections of the tape (32) with a smaller width.

15. The method according to one of the claims 10 to 14, wherein the cutting (S1a; S1b) of the raw tape is conducted using a LASER cutting process.
